(19) Europäisches Patentamt
European Patent Office
Office européen des brevets

(11) Veröffentlichungsnummer: **0 234 437 B1**

# (12) EUROPÄISCHE PATENTSCHRIFT

(45) Veröffentlichungstag der Patentschrift: **31.10.90**

(51) Int. Cl.[5]: **C09K 19/06**, C07C 69/00, C07C 233/00, C07C 255/00, G02F 1/13

(21) Anmeldenummer: **87102009.5**

(22) Anmeldetag: **13.02.87**

(54) **Chirale Umsetzungsprodukte aus mesogenen Molekülbausteinen und bifunktionell reaktionsfähigen Weinsäurederivaten und ihre Verwendung als Dotierstoff in Flüssigkristall-Phasen.**

(30) Priorität: **17.02.86 DE 3604898**

(43) Veröffentlichungstag der Anmeldung: **02.09.87 Patentblatt 87/36**

(45) Bekanntmachung des Hinweises auf die Patenterteilung: **31.10.90 Patentblatt 90/44**

(84) Benannte Vertragsstaaten: **CH DE FR GB LI**

(56) Entgegenhaltungen: **DE-A- 3 333 677**

(73) Patentinhaber: **HOECHST AKTIENGESELLSCHAFT, Postfach 80 03 20, D-6230 Frankfurt am Main 80(DE)**

(72) Erfinder: **Scherowsky, Günter, Prof. Dr., Winklerstrasse 18B, D-1000 Berlin 33(DE)**
Erfinder: **Gunaratne, Manel, Fraunhoferstrasse 26, D-1000 Berlin 10(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 234 437 B1

**Beschreibung**

Die Kennlinien der in Flüssigkristall-Displays verwendeten elektro-optischen Effekt verändern sich im allgemeinen mit der Temperatur. Insbesondere bei einer Ansteuerung im Multiplexbetrieb ergeben sich daraus Schwierigkeiten, die zu einer unerwünschten Einschränkung des Arbeitstemperaturbereiches führen können. Bei verschiedenen elektrooptischen Effekten kann durch Zusatz chiraler Verbindungen zum nematischen Flüssigkristall über die Temperaturfunktion der Ganghöhe der dadurch induzierten cholesterischen Helixstruktur die Temperaturabhängigkeit der elektrooptischen Kennlinien vorteilhaft beeinflußt werden, so beim cholesterisch-nematischen Phasenumwandlungseffekt, der TN("twisted nematic")-Zelle und dem kürzlich vorgestellten SBE ("supertwisted birefringence effect"). Die üblichen bekannten Dotierstoffe induzieren im allgemeinen eine mit zunehmender Temperatur ansteigende Ganghöhe; es sind in jüngster Zeit auch bereits Dotierstoffe beschrieben worden, die diesen oftmals unerwünschten Effekt nicht zeigen.

Aus der DE-C 2 827 471 (= US-A 4 264 148) ist der Zusatz von zwei unterschiedlichen chiralen Dotierstoffen zu nematischen Trägersubstanzen bekannt; dabei erzeugt der eine chirale Dotierstoff in der nematischen Trägersubstanz eine rechtshändige Verdrillung, der andere eine linkshändige Verdrillung. Mit einer solchen Dotierung wird eine Abnahme der Ganghöhe erreicht, aber es sind zur Erreichung dieses Effekts relativ hohe Gesamtkonzentrationen erforderlich, die zu einer negativen Beeinflussung der anderen Materialparameter führen können.

In der DE-A 3 333 677 werden u.a. Umsetzungsprodukte (Ester) von chiralem Butandiol-(2,3) mit mesogenen Carbonsäuren beschrieben, die bereits in Einzeldosierung in Flüssigkristall-Phasen die Optimierung der Temperaturkompensation vereinfachen können. Diese bekannten Ester weisen aber oftmals ein für bestimmte Anwendungen noch zu niedriges Verdrillungsvermögen auf.

Aufgabe der vorliegenden Erfindung ist es deshalb, neue Verbindungen aufzufinden, die bei ihrem Einsatz als chirale Dotierstoffe in Flüssigkristall-Phasen bei verhältnismäßig geringen Zusatzmengen einzeln oder im Gemisch bereits eine Optimierung der Temperaturkompensation und gleichzeitig eine starke Verdrillung der Flüssigkristall-Phasen bewirken; außerdem soll es möglich sein, ausgehend von einer vergleichbaren Grundstruktur, durch kleinere Molekülvariationen das Molekül in seinen Eigenschaften in einer bestimmten Richtung zu verändern.

Die Erfindung geht aus von den bekannten Verbindungen aus einem Molekülbaustein mit zwei Chiralitätszentren und mindestens einem mesogenen Molekülbaustein. Die erfindungsgemäßen Verbindungen sind gekennzeichnet durch die allgemeine Formel (I)

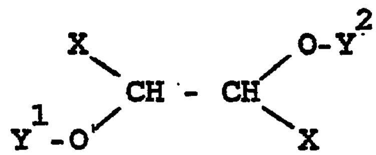

(I)

in der die Symbole folgende Bedeutung haben:

$Y^1$ = H, $(C_1–C_{10})$Alkyl, MC–$CH_2$ oder MC–CO, wobei MC eine mesogene Gruppe der allgemeinen Formel (II) bedeutet

$$R^4–(A^1–)_{n1}(B–)_{n2}(A^2–)_{n3} \qquad (II),$$

$R^4$ = ein geradkettiges oder verzweigtes $(C_1–C_{12})$Alkyl, wobei eine $CH_2$-Gruppe oder ein O-Atom ersetzt sein kann, oder falls n1 = 1 auch F, Cl, Br oder CN

$A^1$, $A^2$ = unabhängig voneinander 1,4-Phenylen, Diazin-2,5-diyl, Diazin-3,6-diyl, 1,4-Cyclohexylen,

B = CO–O, O–CO, $CH_2–CH_2$, $OCH_2$, $CH_2O$,

n1, n2, n3 = unabhängig voneinander 0, 1 oder 2, wobei n1 und n3 nicht gleichzeitig 0 sind,

$Y^2$ = $(C_1–C_{10})$Alkyl, MC–$CH_2$ oder MC–CO, wobei $Y^1$ und $Y^2$ gemeinsam auch eine MC–CH Gruppe darstellen können, die dann Teil eines Dioxolan-Ringes ist,

X = $COOR^1$, $CONH_2$, $CONR^2R^3$ oder C≡N,

$R^1$ = $(C_1–C_{10})$Alkyl oder MC–$CH_2$,

$R^2$, $R^3$ = H und $(C_1–C_4)$Alkyl oder unabhängig voneinander $(C_1–C_4)$Alkyl.

Die genannten Verbindungen sind entweder ein- oder zweifach an den OH-Gruppen verestert oder veretherte Weinsäurediester, gegebenenfalls substituierte Weinsäurediamine oder Weinsäuredinitrile.

Eine weitere Lösung der gestellten Aufgabe ist eine verdrillbare Flüssigkristall-Phase mit einem Gehalt an mindestens einer chiralen Verbindung, die dadurch gekennzeichnet ist, daß sie als chirale Verbindung mindestens eine Verbindung der allgemeinen Formel (I) enthält. Unter dem Begriff "verdrillbare Flüssigkristall-Phase" sind nematische, chloresterische, geneigt("tilted")-smektische, insbesondere smektisch C ($S_c$ oder SmC), Phasen zu verstehen.

Die erfindungsgemäßen verdrillbaren Flüssigkristallphasen bestehen aus 2 bis 20, vorzugsweise 2 bis 15 Komponenten, darunter mindestens einem der erfindungsgemäß beanspruchten chiralen Dotierstoffe. Die anderen Bestandteile werden vorzugsweise ausgewählt aus den bekannten Verbindungen mit nematischen, cholesterischen und/oder geneigt-smektischen Phasen, dazu gehören beispielsweise Schiffsche Basen, Biphenyle, Terphenyle, Phenylcyclohexane, Cyclohexylbiphenyle, Pyrimidine, Zimtsäureester, Cholesterinester, verschieden überbrückte, terminal-polare mehrkernige Ester von p-Alkylbenzoesäuren. Im allgemeinen liegen die im Handel erhältlichen Flüssigkristall-Phasen bereits vor der Zugabe des chiralen Dotierstoffes als Gemische verschiedenster Komponenten vor, von denen mindestens eine mesogen ist, d.h. als Verbindung, in derivatisierter Form oder im Gemisch mit bestimmten Cokomponenten eine Flüssigkristall-Phase zeigt [= mindestens eine enantiotrope (Klärtemperatur > Schmelztemperatur) oder monotrope (Klärtemperatur < Schmelztemperatur) Mesophasenbildung erwarten läßt].

Mit Hilfe der neu-entwickelten Verbindungen als Dotierstoff gelingt es bei geringer Menge an Dotierstoff in Flüssigkristall-Phasen eine hohe Verdrillung zu erzielen, wobei die Verbindungen einzeln oder im Gemisch außerdem eine bei Temperaturänderung im wesentlichen unabhängige Ganghöhe aufweisen können, d.h. die Zu- oder Abnahme der Ganghöhe liegt im allgemeinen im Bereich von 1% bis 1‰ pro K. Zu den besonderen Eigenschaften der erfindungsgemäßen Verbindungen zählt außerdem, daß das Vorzeichen der Temperaturabhängigkeit des Verdrillungsvermögens ($d\beta/dT$) unabhängig vom Vorzeichen der Ganghöhe der induzierten Helixstruktur ist. Durch die geeignete Variation der mesogenen Reste läßt sich entweder eine rechts- oder linkshändige Helix induzieren oder sogar bei einer bestimmten Temperatur eine Helixinversion erreichen; d.h. diese Verbindungen zeichnen sich dadurch aus, daß sich bei einer bestimmten Inversionstemperatur die Händigkeit der Verdrillung verändert, wobei im vorliegenden Fall diese Inversionstemperatur zwischen dem Erstarrungspunkt und dem Klärpunkt der jeweiligen Flüssigkristallphase liegt; bei Verwendung handelsüblicher Flüssigkristallphasen bedeutet dies einen Temperaturbereich von insbesondere −40°C bis +200°C, bevorzugt −20°C bis +140°C. Ein weiterer Einsatz der erfindungsgemäßen Verbindungen kann bei der Thermotopographie oder zur Erzeugung von "blue phases" (= cholesterische Systeme mit relativ kleiner Ganghöhe von z.B. weniger als 800 nm) erfolgen.

Geht man bei der Synthese der genannten Weinsäurederivate statt von der R,R-Form von der S,S-Form aus, so werden an der Temperaturachse gespiegelte Kurvenverläufe der Temperaturabhängigkeit des Verdrillungsvermögens beobachtet, d.h. das Vorzeichen des Helixdrehsinns ist vertauscht. Werden im Molekül die mesogenen Acylreste zu Benzylresten umgewandelt (>C=O zu $>CH_2$), so tritt eine Steigerung des Verdrillungsvermögens der HTP bzw. MTP ein, wobei diese und andere vor- und nachstehend verwendete Begriffe wie folgt definiert sind:

– HTP ("helical twisting power") = 1/p • c, (p = Ganghöhe der induzierten Helixstruktur in µm, c = Konzentration des chiralen Dotierstoffes in Gew.-%),
– MTP ("molecular twisting power"): $\beta = 1/p \cdot N_v$ (p = Ganghöhe in m, $N_v$ = Konzentration des chiralen Dotierstoffes in $mol/m^3$).

Werden die Estergruppierungen auch in Nitrilgruppen umgewandelt, so ändert sich das Vorzeichen des Helixdrehsinns. Darüber hinaus zeigt das Dinitril eine sehr gute spontane Polarisation $P_s$ von 40 $nC \cdot cm^{-2}$ (extrapoliert auf die reine Verbindung), d.h. es ist ein geeigneter Zusatz für die Umwandlung von SmC-Phasen in ferroelektrische Flüssigkristall-Phasen, wobei diese $P_s$ umso besser ist, desto höher die Werte in $nC \cdot cm^{-2}$ sind.

Unter den Verbindungen der allgemeinen Formel (I) sind die bevorzugt, bei denen die Symbole folgende Bedeutung haben: R4 = geradkettiges ($C_4$–$C_{10}$)Alkyl, wobei eine $CH_2$-Gruppe durch ein O-Atom ersetzt sein kann, $A^1$, $A^2$ = unabhängig voneinander unsubstituiertes 1,4-Phenylen, 1,4-Cyclohexylen oder Diazin-2,5-diyl, B = CO–O oder O–CO, n1 = 1, n2 = 0 oder 1, und n3 = 1 oder 2.

Es sind aber auch Reste MC möglich, die sich von chiralen Carbonsäuren wie substituierten Phenylessigsäuren ableiten, z.B. von der 3,3,3-Trifluor-2-methoxy-2-phenylpropansäure.

In der allgemeinen Formel (I) werden darüber hinaus die Verbindungen bevorzugt, bei denen die Symbole folgende Bedeutung haben: $Y^1 = Y^2$ = MC–CO oder MC–$CH_2$ und X = $COOR^1$ mit $R^1$ = ($C_1$–$C_8$)Alkyl, $CONH_2$ oder C ≡ N.

Von der oder den erfindungsgemäßen Dotierstoffen enthalten die Flüssigkristall-Phasen im allgemeinen 0,01 bis 70 Gew.-%, insbesondere 0,05 bis 50 Gew.-%

## Beispiele

Allgemeine Arbeitsvorschrift zur Herstellung der Verbindungen 1 bis 10

Zu 1 mmol des Weinsäurederivates in 10 bis 50 ml wasserfreiem Methylenchlorid oder Dimethylformamid werden unter Rühren 10 bis 40 mg Dimethylaminopyridin und 1,5 mmol der mesogenen Carbonsäure zugegeben. Bei einer Temperatur von 0°C werden 1,5 mmol Dicyclohexylcarbodiimid hinzugefügt, und es wird während 10 min bei dieser Temperatur und dann 20 h bei Raumtemperatur gerührt. Es wird von ausgefallenem Harnstoff abfiltriert, das Filtrat wird im Vakuum eingedampft und der verbleibende Rückstand wird in Methylenchlorid aufgenommen. Nach eventueller Filtration wird das organische Lösemittel

abgezogen und der Rückstand an Kieselgel chromatographiert. Nach dieser Vorschrift werden folgende Verbindungen synthetisiert, deren Struktur durch spektroskopische Daten und Elementaranalysen gesichert sind:

(Ausgehend von dem R,R-Weinsäurediester)

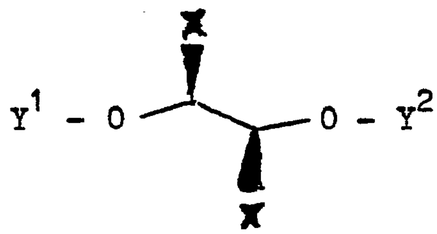

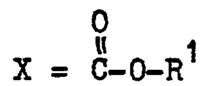

(1) $R^1 = CH_3$ $Y^1 = Y^2$:

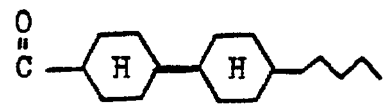

R,R-(–)-2,3-Bis-(4′-trans-n-pentyl-4-trans-dicyclohexyl-carbonyloxy)-butan-1,4-disäure-dimethylester

(2) $R^1 = CH_3$ $Y^1 = Y^2$:

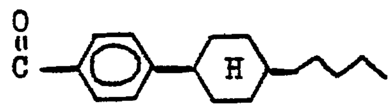

R,R-(–)-2,3-Bis-[4-(trans-4-n-pentyl-cyclohexyl)-benzoyloxy]-butan-1,4-disäure-dimethylester

(3) $R^1 = C_2H_5$ $Y^1 = Y^2$:

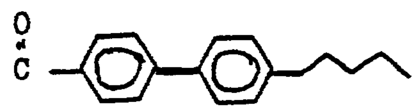

R,R-(–)-2,3-Bis-(4′-n-pentyl-4-diphenylcarbonyloxy)-butan-1,4-disäure-diethylester

(4) $R^1 = CH_3$ $Y^1 = Y^2$:

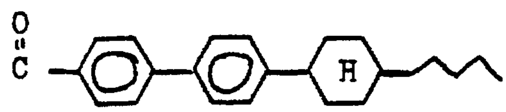

R,R-2,3-Bis-(4′-trans-n-pentyl-cyclohexyl-4-diphenyl-carbonyloxy)-butan-1,4-disäure-dimethylester

(5) $R^1 = CH_3$ $Y^1 = Y^2$:

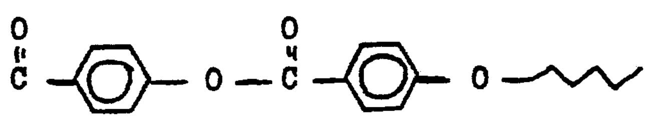

R,R-(–)-2,3-Bis-[4-(4-n-hexyloxy-benzoyloxy)-benzoyloxy]-butan-1,4-disäure-dimethylester

(6) $R^1 = CH_3$ $Y^1 = H$

$Y^2$ = 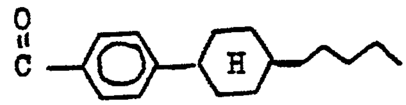

R,R-(+)-2-[4-(trans-4-n-Pentyl-cyclohexyl)-benzoyloxy]-3-hydroxybutan-1,4-disäure-dimethylester
(7) $R^1 = CH_3$ $Y^1 = H$

$Y^2$ = 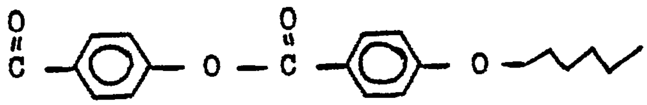

R,R-(+)-2-[4-(4-n-Hexyloxy-benzoyloxy)-benzoyloxy]-3-hydroxy-butan-1,4-disäure-dimethylester
(8) $R^1 = C_2H_5$ $Y^1 = Y^2$:

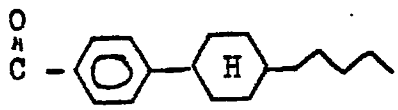

R,R-(–)-2,3-Bis-[4-(trans-4-n-pentyl-cyclohexyl)-benzoyloxy]-butan-1,4-disäure-diethylester
(9) $R^1 = C_8H_{17}$ $Y^1 = Y^2$:

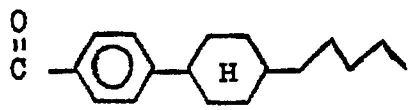

R,R-2,3-Bis-[4-(trans-4-n-pentyl-cyclohexyl)-benzoyloxy]-butan-1,4-disäure-di-n-octylester
(Ausgehend von dem S,S-Weinsäurediester)

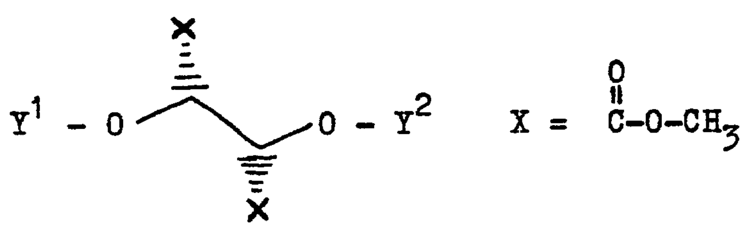

(10) $Y^1 = Y^2$:

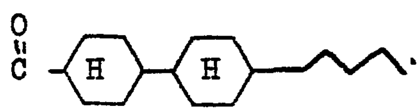

S,S-2,3-Bis-(4′-trans-n-pentyl-4-trans-dicyclohexylcarbonyloxy)-butan-1,4-disäure-dimethylester

Herstellung der Verbindung 11

Zur Lösung von 10 mmol RR-Weinsäurediethylester in 7 ml Acetonitril werden zunächst 20 mmol Thalliumethylat und dann 27 mmol 4-(trans-4-n-Pentylcyclohexyl)-benzylbromid so langsam zugegeben, daß keine Erwärmung eintritt. Anschließend wird das Reaktionsgemisch während 50 h auf 58°C erwärmt und noch 14 h bei Raumtemperatur gerührt. Der entstandene Thalliumbromid-Niederschlag wird abfiltriert, das organische Lösemittel im Vakuum entfernt und der verbleibende Rückstand an Kieselgel chromatographiert.

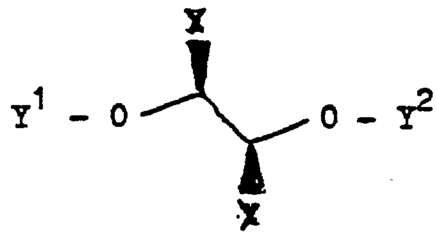

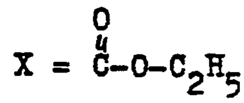

(11) $Y^1 = Y^2$:

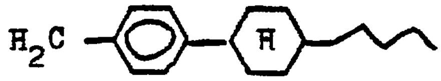

R,R-2,3-Bis-[4-(trans-4-n-pentyl-cyclohexyl)-benzyloxy]-butan-1,4-disäure-diethylester

Herstellung der Verbindungen 12 und 13

Es wird nach der weiter vorne stehenden allgemeinen Arbeitsvorschrift verfahren, aber ausgehend von der R,R-(+)-2,3-Dimethoxybutan-1,4-disäure [siehe D. Seebach, Helv. Chim. Acta 60, 301 (1977)] und dem entsprechenden Alkohol.

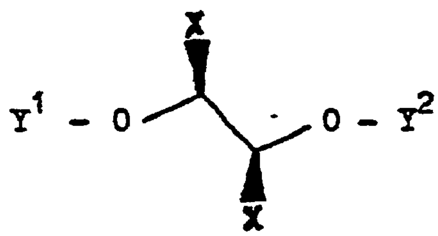

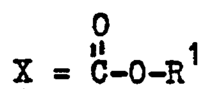

$Y^1 = Y^2 = CH_3$

(12)

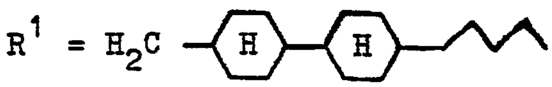

R,R-2,3-Dimethoxy-butan-1,4-disäure-di(4-trans-n-pentyl-4-trans-dicyclohexyl-methylester)

(13)

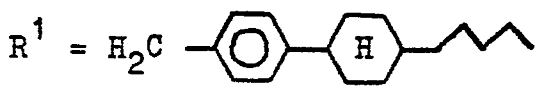

R,R-2,3-Dimethoxy-butan-1,4-disäure-di[4-(trans-4-n-pentyl-cyclohexyl)-benzylester]

Herstellung der Verbindung 14

Eine Mischung von 0,5 g (1,5 mmol) des 4(trans-4-n-Pentyl-cyclohexyl)-4-biphenylcarbaldehyds, 0,53 g R,R-Weinsäuredimethylester, 0,03 g der p-Toluolsulfonsäure • $H_2O$ wird in 20 ml Cyclohexan bei 0°C gerührt und ein unter Cyclohexan feingepulvertes Molekularsieb (0,5 µm) zugegeben. Die Mischung wird während 2 h bei 0°C und über Nacht bei Raumtemperatur gerührt, dann werden 0,05 ml Trimethylamin zugesetzt. Die Suspension wird filtriert, das Molekularsieb mit Pentan gewaschen, und die vereinigten organischen Phasen werden mit 15%iger wäßriger NaOH-Lösung und danach mit $H_2O$ gewaschen. Die organische Phase wird getrocknet und eingedampft.

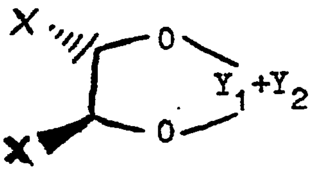

$Y^1 + Y^2$ gemeinsam = MC-CH

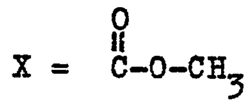

(14) $Y^1 + Y^2$ gemeinsam:

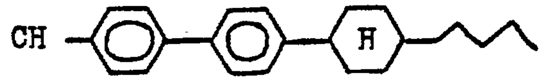

R,R-2-[4-(trans-4-n-Pentyl-cyclohexyl)-biphenyl]-1,3-dioxolan-4,5-dicarbonsäure-dimethylester

Herstellung der Verbindung 15

Zu einer Lösung von 0,5 mmol der Verbindung 11 in 2 ml Chloroform werden bei –20°C etwa 3 ml $NH_3$-gesättigter Methanol gegeben. Die Lösung wird während 3 Tagen bei Raumtemperatur gerührt. Der entstandene Niederschlag wird abfiltriert und umkristallisiert.

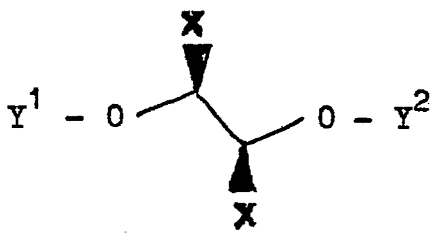

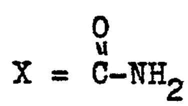

(15) $Y^1 = Y^2$:

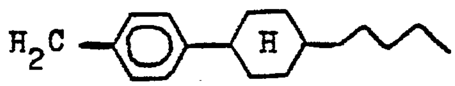

R,R-2,3-Bis-[4-(trans-4-n-pentyl-cyclohexyl)-benzyloxy]-butan-1,4-disäurediamid

Herstellung der Verbindung 16

Eine Lösung von 0,3 mmol der Verbindung 15 in 2 ml Pyridin wird auf 0°C gekühlt und es werden 0,6 mmol $POCl_3$ zugetropft, danach wird während 1 h bei 0°C und 1 h bei Raumtemperatur gerührt. Nach erneuter Kühlung werden 4 ml $H_2O$ zugegeben, der ausgefallene Niederschlag wird danach mit Wasser gewaschen und in Ether aufgenommen. Nach Abdestillieren vom organischen Lösemittel wird der Rückstand an Kieselgel chromatographiert.

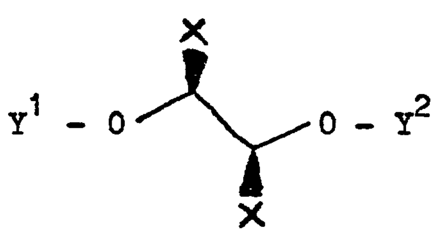

X = C≡N

(16) $Y^1 = Y^2$:

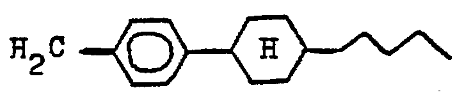

R,R-2,3-Bis-[4-(trans-4-n-pentyl-cyclohexyl)-benzyloxy]-butan-1,4-disäuredinitril

Tabelle

| Verbindungs-Nr. | Temperaturintervall der Messung des Verdrillungsvermögens (°C) | p·c (µm·Gew.-%) | Schmelzpunkt (°C) |
|---|---|---|---|
| 1 | 7 – 103 | 6 $\leftarrow$ 13 | 97 |
| 2 | 3 – 98 | 101 $\rightarrow$ -40 | 74 |
| 3 | 10 – 100 | -22 $\rightarrow$ -16 | flüssig |
| 4 | 4 – 100 | -51 $\rightarrow$ -16 | – |
| 5 | 10 – 100 | 10 $\rightarrow$ 13 | 105 – 106 |
| 6 | – | – | 45 – 50 |
| 7 | 10 – 100 | -29 $\rightarrow$ -16 | 90 |
| 8 | 10 – 100 | 31 $\rightarrow$ -99 | 48 – 50 |
| 9 | 10 – 100 | 39 $\rightarrow$ -136 | 38 – 40 |
| 10 | – | – | 99 – 101 |
| 11 | 10 – 100 | 4,5 $\rightarrow$ 5,7 | 70 – 73 |
| 12 | 10 – 100 | 9 $\rightarrow$ 18 | 120 – 122 |
| 13 | 10 – 100 | 6 $\rightarrow$ 14 | 74 – 76 |
| 14 | – | – | 110 – 112 |
| 15 | – | – | 212 – 213 |
| 16 | 10 – 100 | -16 $\rightarrow$ -25 | 154 – 156 |

Die Messung des Verdrillungsvermögens wird in einer handelsüblichen nematischen Weitbereichsmischung – "RO-TN 404" der Hoffmann-La Roche Aktiengesellschaft (Basel/Schweiz) – mit einem Klärpunkt von 104°C durchgeführt. Die spontane Polarisation (Ps) – soweit meßbar – wird in der SmC-Phase der handelsüblichen Verbindung "HEPTOAB" (Hersteller z.B. Frinton – USA) mit den Kenndaten "K 74,4 SmC 95,4 N 142,2 I" bestimmt.

In der anliegenden Zeichnung sind die HTP-Werte (Fig. 1) in Abhängigkeit von der Temperatur für verschiedene der erfindungsgemäßen Verbindungen aufgetragen und die MTP-Werte (Fig. 2) für ebenfalls einige Verbindungen.

Vergleicht man in Fig. 1 beispielsweise den Kurvenverlauf der HTP-Werte der Verbindungen 1 bis 4 und 12 miteinander, so läßt sich entweder eine links- oder rechtshändige Helix induzieren oder es findet eine Helixinversion statt, je nachdem welcher Art der mesogene Rest ist. Vergleicht man den entsprechenden Kurvenverlauf der Verbindungen 5 und 7, so zeigt sich, daß sich beim Übergang vom Monoacylderivat 7 zum Diacylderivat 5 die Händigkeit der induzierten Helix ändert, das Vorzeichen der Temperaturabhängigkeit der HTP jedoch nicht. Vergleicht man dann den entsprechenden Kurverlauf der Verbindungen 2, 11 und 16, so zeigt sich, daß die Umwandlung der Acyl- zu einer Benzylgruppen zu einer starken Steigerung der HTP führt und der Ersatz der Ester- in Nitrilgruppen wieder die Händigkeit der Helix verändert. Fig. 2 zeigt, daß bei gleichem Aufbau der mesogenen Reste die Verlängerung des Alkylrestes in den Esterfunktionen von $CH_3$ zu $C_2H_5$ zwar zu einer starken Veränderung der Inversionstemperatur führt, bei einer Verlängerung auf $C_8H_{17}$ sich dann aber nur noch gering verändert.

## Patentansprüche

1. Verbindungen aus einem Molekülbaustein mit zwei Chiralitätszentren und mindestens einem mesogenen Molekülbaustein gemäß der allgemeinen Formel (I),

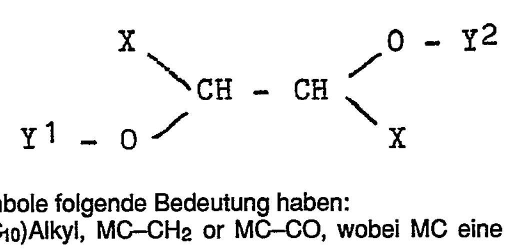

(I)

in der die Symbole folgende Bedeutung haben:
$Y^1$ = H, $(C_1–C_{10})$Alkyl, MC–$CH_2$ or MC–CO, wobei MC eine mesogene Gruppe der allgemeinen Formel (II) bedeutet

$$R^4\text{–}(A^1\text{–})_{n1}(B\text{–})_{n2}(A^2\text{–})_{n3} \qquad (II)$$

in der die Symbole folgende Bedeutung haben:
$R^4$ = ein geradkettiges oder verzweigtes ($C_1$–$C_{12}$)Alkyl wobei eine $CH_2$-Gruppe durch ein O-Atom ersetzt sein kann, oder falls n1 = 1 auch F, Cl, Br oder CN
$A^1$, $A^2$ = unabhängig voneinander 1,4-Phenylen, Diazin-2,5-diyl, Diazin-3,6-diyl, 1,4-Cyclohexylen,
B = CO–O, O–CO, $CH_2$–$CH_2$, $OCH_2$, $CH_2O$,
n1, n2, n3 = unabhängig voneinander 0, 1 oder 2, wobei n1 und n3 nicht gleichzeitig 0 sind,
$Y^2$ = ($C_1$–$C_{10}$)Alkyl, MC–$CH_2$ oder MC–CO, wobei $Y^1$ und $Y^2$ gemeinsam auch eine MC–CH Gruppe darstellen können, die dann Teil eines Dioxolan-Rings ist,
X = $COOR^1$, $CONH_2$, $CONR^2R^3$ oder C≡N,
$R^1$ = ($C_1$–$C_{10}$)Alkyl oder MC–$CH_2$ und
$R^2$, $R^3$ = H und ($C_1$–$C_4$)Alkyl oder unabhängig voneinander ($C_1$–$C_4$)Alkyl bedeuten.

2. Verdrillbare Flüssigkristall-Phase mit einem Gehalt an mindestens einer chiralen Verbindung der allgemeinen Formel (I) nach Anspruch 1.

3. Flüssigkristall-Phase nach Anspruch 2, dadurch gekennzeichnet, daß sie 0,01 bis 70 Gew.-% an mindestens einer der chiralen Verbindungen enthält.

4. Flüssigkristall-Anzeigeelement enthaltend eine Flüssigkristall-Phase nach Anspruch 2.

5. Verwendung einer chiralen Verbindung gemäß der allgemeinen Formel (I) nach Anspruch 1 zur Temperaturkompensation und Erzeugung einer Verdrillung in Flüssigkristall-Phasen.

6. Verfahren zur Temperaturkompensation und Verdrillung in Flüssigkristall-Anzeigeelementen, die eine Flüssigkristall-Phase enthalten, durch Zusatz von mindestens einer chiralen Verbindung, dadurch gekennzeichnet, daß man der Flüssigkristall-Phase 0,01 bis 70 Gew.-% mindestens eine Verbindung gemäß der allgemeinen Formel (I) nach Anspruch 1 zusetzt.

## Claims

1. A compound comprising a molecular structural element with two chirality centers and at least one mesogenic molecular structural element according to the formula (I)

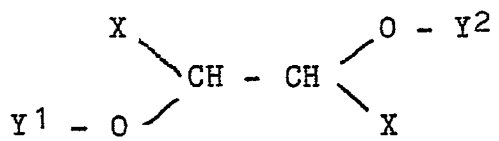

(I)

in which the symbols have the following meanings:
$Y^1$ = H, ($C_1$–$C_{10}$)alkyl, MC–$CH_2$ or MC–CO, MC denoting a mesogenic group of the formula (II)

$$R^4\text{–}(A^1\text{–})_{n1}(B\text{–})_{n2}(A^2\text{–})_{n3} \qquad (II)$$

in which the symbols have the following meanings:
$R^4$ = a straight-chain or branched ($C_1$–$C_{12}$)alkyl, it being possible for a $CH_2$ group to be replaced by an O atom, or if n1 = 1 also F, Cl, Br or CN,
$A^1$, $A^2$ = independently of each other, 1,4-phenylene, diazine-2,5-diyl, diazine-3,6-diyl or 1,4-cyclohexylene,
B = CO–O, O–CO, $CH_2$–$CH_2$, $OCH_2$, $CH_2O$,
n1, n2, n3 = independently of each other, 0, 1 or 2, n1 and n3 not being 0 at the same time,
$Y^2$ = ($C_1$–$C_{10}$)alkyl, MC–$CH_2$ or MC–CO, it being possible for $Y^1$ and $Y^2$ to jointly represent also a MC–CH group, which is then part of a dioxolane ring,
X = $COOR^1$, $CONH_2$, $CONR^2R^3$ or C ≡ N,
$R^1$ = ($C_1$–$C_{10}$)alkyl or MC–$CH_2$ and
$R^2$, $R^3$ = H and ($C_1$–$C_4$)alkyl or, independently of each other, ($C_1$–$C_4$)alkyl.

2. A twistable liquid crystal phase containing at least one chiral compound of the formula (I) as claimed in claim 1.

3. A liquid crystal phase as claimed in claim 2, wherein the phase contains 0.01 to 70% by weight of at least one of the chiral compounds.

4. A liquid crystal display element containing a liquid crystal phase as claimed in claim 2.

5. The use of a chiral compound according to the formula (I) as claimed in claim 1 for temperature compensation and producing twisting in liquid crystal phases.

6. A method for temperature compensation and twisting in liquid crystal display elements, which contain a liquid crystal phase, by adding at least one chiral compound, wherein 0.01 to 70% by weight of at least one compound according to the formula (I) as claimed in claim 1 is added to the liquid crystal phase.

## Revendications

1. Composés constitués d'un motif moléculaire comportant deux centres de chiralité et au moins un composant moléculaire mésogène selon la formule générale (I),

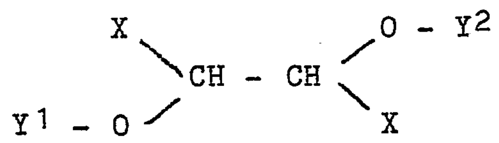

(I)

où les symboles ont les significations suivantes:
$Y^1$ = H, un radical alkyle en $C_1$–$C_{10}$, MC–$CH_2$ ou MC–CO, où MC est un groupe mésogène de formule générale (II)

$$R^4\text{–}(A^1)_{n1}(B\text{–})_{n2}(A^2\text{–})_{n3} \qquad \text{(II)},$$

$R^4$ est un radical alkyle en $C_1$ à $C_{12}$ à chaîne droite ou ramifiée, un groupe $CH_2$ pouvant être remplacé par un atome d'oxygène, ou encore, si n1 = 1, peut être aussi F, Cl, Br ou CN,
$A^1$ et $A^2$, indépendamment l'un de l'autre, représentent chacun un radical phénylène-1,4, diazinediyle-2,5 diazinediyle-3,6, cyclohexylène-1,4,
B = CO–O, O–CO, $CH_2$–$CH_2$, $OCH_2$, $CH_2O$,
n1, n2, n3, indépendamment les uns des autres, valent chacun 0, 1 ou 2, n1 et n3 ne valant pas simultanément 0,
$Y^2$ est un radical alkyle en $C_1$–$C_{10}$, MC–$CH_2$ ou MC–CO, où $Y^1$ et $Y^2$ peuvent ensemble représenter aussi un groupe MC–CH, qui fait alors partie d'un cycle dioxolanne,
X = $COOR^1$, $CONH_2$, $CONR^2R^3$ ou C..N,
$R^1$ est un radical alkyle en $C_1$–$C_{10}$ ou MC–$CH_2$,
$R^2$, $R^3$ = H et un radical alkyle en $C_1$–$C_4$, ou représentent chacun, indépendamment l'un de l'autre, un radical alkyle en $C_1$–$C_4$.

2. Phase mésomorphe torsadable contenant au moins un composé chiral de formule générale (I) selon la revendication 1.

3. Phase mésomorphe selon la revendication 2, caractérisée en ce qu'elle contient de 0,01 à 70% en poids d'au moins l'un des composés chiraux.

4. Elément d'affichage à cristaux liquides contenant une phase mésomorphe selon la revendication 2.

5. Utilisation d'un composé chiral selon la formule générale (I) de la revendication 1 pour la compensation des températures et la production d'un torsadage dans des phases mésomorphes.

6. Procédé de compensation des températures et de torsadage dans des éléments d'affichage à cristaux liquides contenant une phase mésomorphe, par addition d'au moins un composé chiral, caractérisé en ce qu'on ajoute à la phase mésomorphe de 0,01 à 70% en poids d'au moins un composé selon la formule générale (I) de la revendication 1.

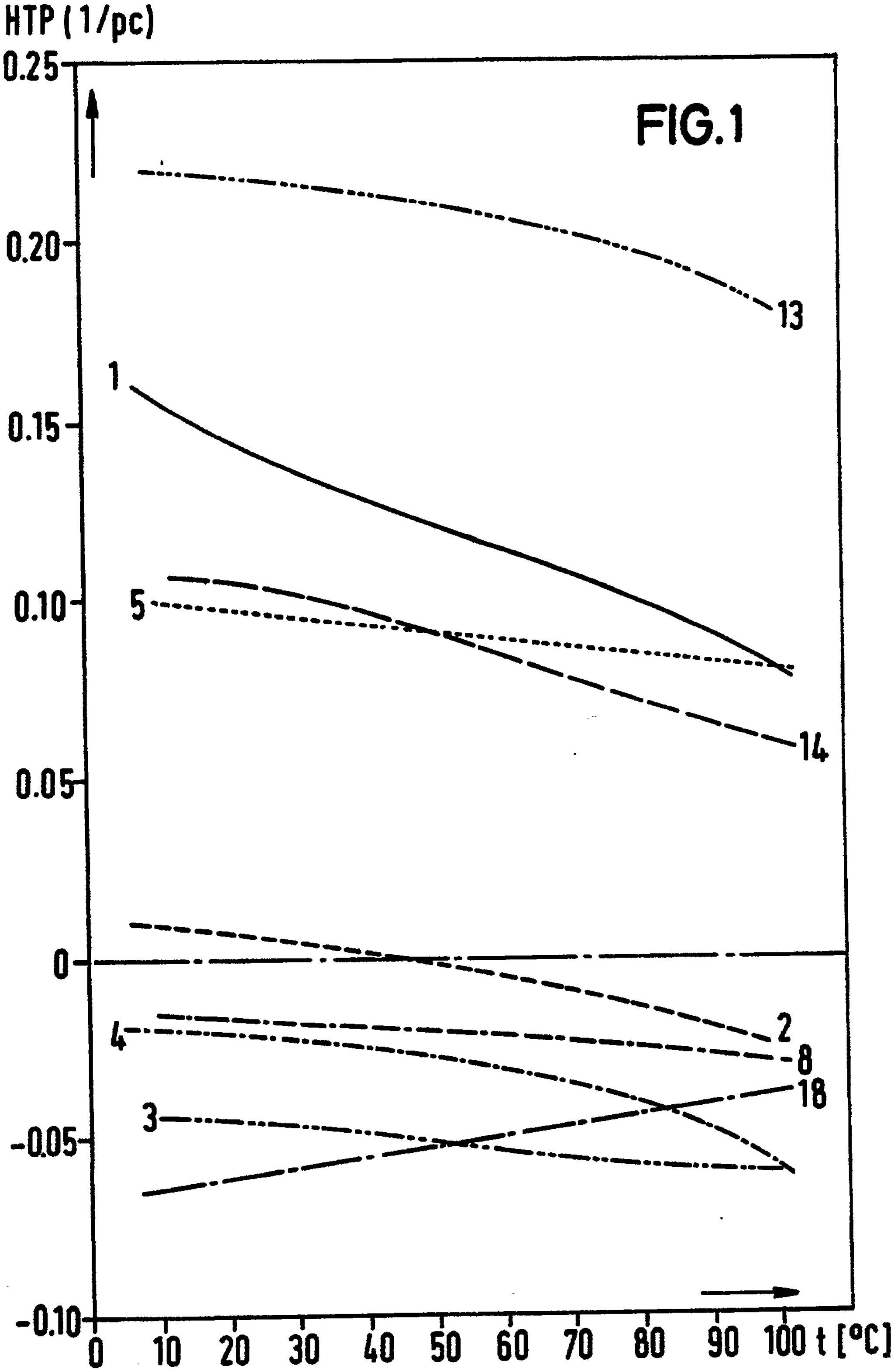
HTP (1/pc)
0.25
0.20
0.15
0.10
0.05
0
-0.05
-0.10
FIG.1
13
1
5
14
2
8
18
4
3
0
10
20
30
40
50
60
70
80
90
100
t [°C]

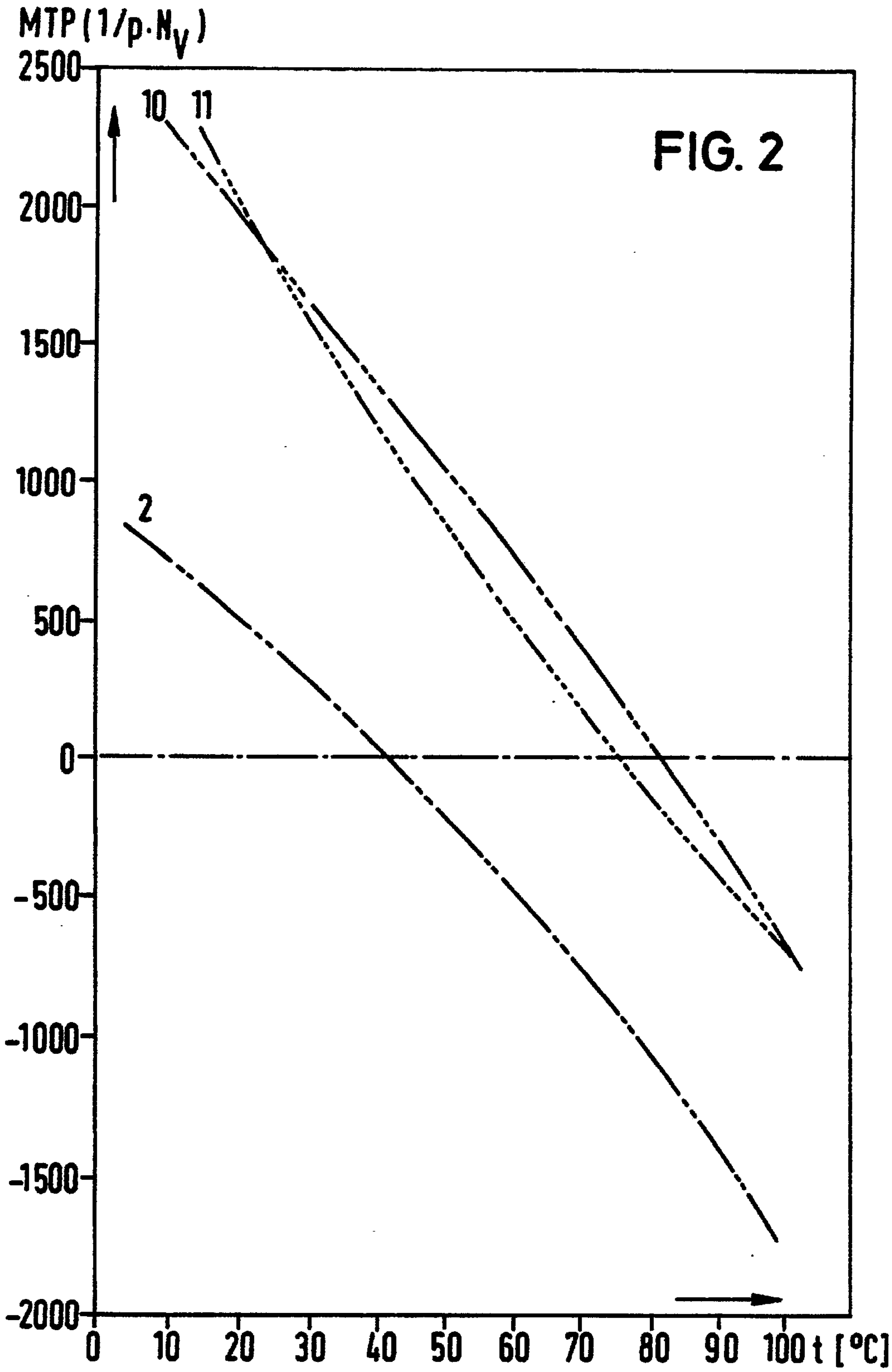
MTP (1/p·$N_V$)
2500
2000
1500
1000
500
0
-500
-1000
-1500
-2000
10
11
2
0
10
20
30
40
50
60
70
80
90
100
t [°C]

FIG. 2